# FASCICULE DE BREVET EUROPEEN

(11) **EP 0 504 066 B1**
(45) Date de publication et mention de la délivrance du brevet: **04.09.1996**
(21) Numéro de dépôt: 92400671.1
(22) Date de dépôt: 13.03.1992
(51) Int. Cl.: A61K 7/48, A61K 7/02, A61K 7/027, A61K 7/043

(54) **Compositions cosmétiques contenant une dispersion de particules solides dont la surface est revêtue à l'aide d'un polymère cationique**
Kosmetische Zusammensetzung, die eine Dispersion fester Teilchen enthält, deren Oberfläche mit einem kationischen Polymer beschichtet ist
Cosmetic compositions containing a dispersion of solid particles surface-coated with a cationic polymer

(30) Priorité: 14.03.1991 FR 9103111
(43) Date de publication de la demande: 16.09.1992
(73) Titulaire: L'OREAL, F-75008 Paris (FR)
(72) Inventeur: Mellul, Myriam, F-94240 L'Hay les Roses (FR); Candau, Didier, F-77000 Melun (FR)
(74) Mandataire: Tonnellier, Jean-Claude

(56) Documents cités:
- EP-A- 0 249 685
- EP-A- 0 279 319
- EP-A- 0 369 741
- FR-A- 2 234 359
- LU-A- 57 903

## Description

La présente invention a pour objet des compositions cosmétiques pour la peau ou les phanères, ou encore des compositions de vernis à ongles, contenant une dispersion de particules solides, dans lesquelles sont introduites des particules solides dont la surface est revêtue à l'aide d'un polymère cationique.

On sait que divers produits de maquillage tels que les poudres libres ou compactées, les fonds de teint, les fards à joues, les fards à paupières, les compositions pour le maquillage des cils et les sourcils (par exemple les mascaras), les compositions pour le maquillage du bord des paupières appelées 'ligneurs" (ou eye liners), ainsi que les rouges à lèvres sont présentés sous la forme de compositions comprenant une dispersion de particules minérales solides dans un liant gras. Il peut s'agir de compositions anhydres, ou bien d'émulsions huile-dans-l'eau ou eau-dans-l'huile.

Selon les types de compositions, les particules solides sont soit uniquement des pigments (blancs et/ou colorés), destinés à conférer à la zone d'application (par exemple peau du visage ou lèvres) une certaine coloration, soit des particules, généralement appelé es "charges", qui ont des fonctions diverses variant avec la nature des particules.

Dans les compositions à appliquer sur la peau, on utilise souvent des charges destinées à apporter un pouvoir couvrant, c'est-à-dire à masquer les imperfections de la peau (différences de coloration, microreliefs) soit grâce à leur opacité (c'est le cas notamment pour les oxydes de titane et de zinc et pour le kaolin) soit par leurs propriétés de réflexion de la lumière (c'est le cas notamment des charges lamellaires telles que le talc et les micas). On utilise également des charges qui sont capables d'absorber les sécrétions aqueuses et huileuses de la peau, afin d'éviter l'aspect luisant de la peau et la migration des matières colorantes : on emploie par exemple à cet effet le kaolin, l'amidon, le carbonate de calcium précipité, la bentonite, etc...

On utilise aussi des particules micronisées ou non de Ti0₂ et de ZnO comme agents d'absorption de l'ultraviolet.

Dans les rouges à lèvres, les particules solides dispersées dans un liant gras approprié sont surtout des pigments colorés, éventuellement en association avec des pigments blancs (par exemple de fines particules de dioxyde de titane) qui permettent de nuancer les teintes apportées par les pigments colorés.

On utilise également de tels pigments blancs et/ou colorés dans les compositions de vernis à ongles, qui sont essentiellement constitués d'une dispersion de ces pigments dans une solution d'un polymère filmogène et d'un plastifiant dans un solvant organique approprié.

La préparation et l'utilisation des compositions cosmétiques contenant des dispersions de particules solides posent plusieurs sortes de problèmes. Un problème commun à la réalisation de l'ensemble des compositions dont on vient de parler réside dans la difficulté d'obtenir des dispersions stables, de façon à appliquer, par exemple sur la peau, un maquillage régulier dont l'application est uniforme et qui conserve une bonne homogénéité. Pour cela, les spécialistes ont été amenés à effectuer des traitements de surface sur les poudres utilisées, notamment afin de modifier les propriétés interfaciales intervenant dans les phénomènes de mouillage et de dispersion. Le but de ces traitements est souvent de rendre la poudre hydrophobe afin de favoriser son incorporation dans les liants et les huiles de formulation, et d'augmenter la stabilité de la dispersion en diminuant les phénomènes de floculation et d'agrégation ; voir par exemple le brevet européen 279 319 qui décrit l'enrobage des pigments par des polymères siliconés.

Ces traitements permettent donc de régler les problèmes de stabilité de la dispersion, en limitant les phénomènes de floculation. Toutefois, ils ne règlent pas un autre problème important, à savoir les faibles propriétés d'adhérence sur la peau des particules solides. En effet, on sait que les particules solides utilisées notamment dans les compositions sous forme de poudres, n'ont que de faibles propriétés d'adhérence sur la peau. Les traitements de surface destinés à améliorer la stabilité des dispersions dans les liants gras n'apportent pas d'amélioration sensible en ce qui concerne les propriétés d'adhérence.

On sait par ailleurs que les produits de maquillage pour le visage et pour les yeux sont souvent présentés sous la forme de poudres compactées. Les poudres compactées sont préparées par mélange des constituants de la poudre avec un agent liant, puis mises sous la forme désirée par compression dans des conteneurs appropriés.

Les poudres compactées doivent présenter des caractéristiques de dureté particulières. La dureté est fonction de la pression de compactage appliquée. Si le produit compacté est trop mou, il sera très fragile et une quantité trop importante de produit sera prélevée au moment de l'application. Par contre, s'il est trop dur, le délitage sera difficile. Par ailleurs, un produit compact doit présenter une surface parfaitement plane. Enfin, il doit répondre favorablement au test de chute, c'est-à-dire présenter une perte de poids réduite après une chute effectuée dans des conditions précises.

On a maintenant découvert qu'il est possible d'obtenir des compositions cosmétiques, comprenant une dispersion de particules solides dans un liant, ayant de bonnes propriétés de stabilité et d'adhérence sur la peau ou sur les phanères, en introduisant dans lesdites compositions des particules solides dont la surface a été revêtue avec un polymère cationique. On a constaté, de façon surprenante que le revêtement des particules solides par des polymères cationiques, qui constituent pourtant un revêtement hydrophile, n'empêche pas l'obtention d'une bonne dispersibilité des particules dans les liants gras. En outre, les compositions compactées obtenues avec des particules solides revêtues de polymère cationique présentent de façon inattendue de bonnes propriétés de cohésion qui se traduisent notamment par un comportement très satisfaisant dans les tests de chute. Par ailleurs, les compositions ainsi obtenues ont de bonnes propriétés d'adhérence, par exemple sur la peau ou les cheveux, après application.

Le document LU-A-57903 décrit des compositions cosmétiques, en particulier des compositions de rouge à lèvres, contenant des pigments micro-encapsulés dans des polymères, sans prévoir l'utilisation de polymères cationiques.

La présente invention a donc pour objet une composition cosmétique pour la peau ou les phanères, comprenant une dispersion de particules solides dans un liant, caractérisée par le fait qu'au moins une partie desdites particules sont introduites dans ladite composition sous la forme de particules dont la surface est revêtue d'au moins un polymère cationique.

Dans les compositions de l'invention, des particules solides sont revêtues en surface avec un polymère cationique. Cela signifie qu'après revêtement, il n'y a ni changement de morphologie ni modification notable des tailles des particules, comme on peut le vérifier par microscopie électronique.

Dans la présente demande, l'expression "polymère cationique" désigne un polymère contenant des groupements cationiques ou des groupements ionisables en groupements cationiques.

Les polymères cationiques préférés sont choisis parmi ceux qui contiennent des motifs comportent des groupements amines primaires, secondaires, tertiaires et/ou quaternaires pouvant soit faire partie de la chaîne polymère soit être portés par un substituant latéral.

Les polymères cationiques utilisés ont de préférence une masse moléculaire comprise entre 10³ et 3.10⁶ environ.

De préférence, les particules revêtues utilisées dans les compositions de l'invention sont revêtues uniquement avec un (ou plusieurs) polymère(s) cationique(s).

Les polymères cationiques utilisés sont notamment ceux qui contiennent au moins 10 % en poids de motifs comportant des groupements amines ou des groupements ammonium quaternaires dont le taux de quaternisation, exprimé en équivalent cationique par gramme de polymère, est par exemple au moins égal à 0,05 méq cationique/g (méq : mil- liéquivalent).

Lorsque le polymère cationique porte des groupements amine ou ammonium quaternaire portés par un substituant latéral, la chaîne polymère est par exemple une channe acrylique, vinylique, siliconée, fluorée ou saccharidique.

Parmi les polymères cationiques on peut citer plus particulièrement les protéines quaternisées, les polysiloxanes quaternisés et les polymères du type polyamine, polyaminoamide et polyammonium quaternaire.

Les quantités de polymère déposées sur les particules varient avec le mode opératoire utilisé pour le revêtement. Généralement, la proportion pondérale de polymère cationique, par rapport au poids total des particules revêtues, est au moins égale à 0,1 % ; la limite supérieure de la quantité de polymère cationique est suffisamment faible pour que les particules gardent leur individualité et leur forme. Autrement dit, le polymère cationique forme, au plus, une couche mince (éventuellement lacunaire) sur les particules revêtues. Le plus souvent, la proportion pondérale de polymère cationique, dans les particules revêtues, est inférieure à 10%, et de préférence inférieure à 8%, par rapport au poids total des particules revêtues.

Les protéines quaternisées sont en particulier des polypeptides modifiés chimiquement portant en bout de chaîne, ou greffés sur celle-ci, des groupements ammonium quaternaires. Parmi ces protéines on peut citer notamment :
- les hydrolysats de collagène portant des groupements triéthylammonium tels que les produits vendus sous la dénomination "QUAT-PRO E"@ par la société MAYBROOK et dénommés dans le dictionnaire CTFA "Triéthonium Hydrolyzed Collagen Ethosulfate";
- les hydrolysats de collagène portant des groupements chlorure de triméthylammonium ou de triméthylstéarylam- monium,vendus sous la dénomination de QUAT-PRO S@ par la société MAYBROOK et dénommés dans le dictionnaire CTFA "Steartrimonium Hydrolyzed Collagen";
- les hydrolysats de protéines animales portant des groupements triméthylbenzylammonium tels que les produits vendus sous la dénomination "CROTEIN BTA"® par la société CRODA et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le CROQUAT L@ dont la chaîne peptidique a un poids moléculaire moyen d'environ 2500 et dont le groupement ammonium quaternaire comporte un groupement alkyle en C₁₂;
- le CROQUAT M® dont la chaîne peptidique a un poids moléculaire moyen d'environ 2500 et dont le groupement ammonium quaternaire comporte un groupement alkyle en C₁₀-C₁₈
- le CROQUAT S® dont la chaîne polypeptidique a un poids moléculaire moyen d'environ 2700 et dont le groupement ammonium quaternaire comporte un groupement alkyle en C₁₈;
- le CROTEIN Q@ dont la chaîne polypeptidique a un poids moléculaire moyen de l'ordre de 12 000 et dont le groupement ammonium quaternaire comporte au moins un groupe alkyle ayant de 1 à 18 atomes de carbone.

Ces différents produits sont vendus par la société CRODA.

D'autres protéines quaternisées sont celles répondant à la formule : 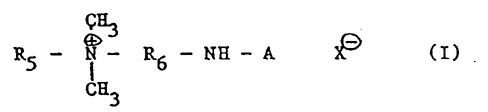 X- est un anion d'un acide organique ou minéral dans laquelle A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone, ces protéines ont un poids moléculaire compris entre 1500 et 10 000 de préférence 2000 et 5000. On peut citer par exemple les produits vendus par la Société INOLEX, sous la dénomination "LEXEIN QX 3000"@, appelé dans le dictionnaire CTFA "Cocotrimonium Collagen Hydrolysate".

Parmi les protéines quaternisées, on peut également citer les protéines végétales quaternisées, telles que les protéines de blé, de maïs ou de soja ; comme protéines de blé quaternisées, on peut citer celles commercialisées par la société CRODA sous les dénominations "HYDROTRITICUM WQ ou QM"@, appelées dans le dictionnaire CTFA "coco- dimonium hydrolysed wheat protein", "HYDROTRITICUM QL"@ appelée dans le dictionnaire CTFA "Laurdimonium hydrolysed wheat protein", ou encore sous la dénomination "HYDROTRITICUM QS"@ appelée dans le dictionnaire CTFA "Steardimonium hydrolysed wheat protein".

Une autre famille de polymères cationiques sont les polymères cationiques siliconés. Parmi ces polymères on peut citer
(a) les polysiloxanes quaternisés dénommés dans le dictionnaire CTFA "Amodiméthicone" et répondant à la formule : 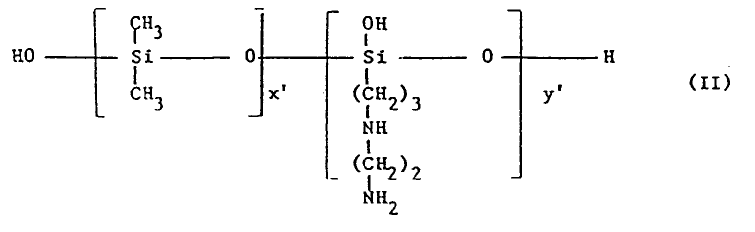 dans laquelle x' et y'sont des nombres entiers dépendant du poids moléculaire qui est compris généralement entre 5000 et 10 000;
(b) les polymères cationiques siliconés répondant à la formule : 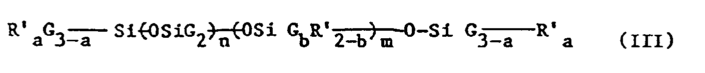 dans laquelle
   G est un atome d'hydrogène, ou le groupement phényle, OH, un groupement alkyle en C₁-C₈ et de préference méthyle,
   a désigne 0 ou un nombre entier de 1 à 3 et de préférence 0,
   b désigne 0 ou 1 et de préference 1,
   la somme (n + m) est un nombre entier de 1 à 2000 et de préférence de 50 à 150, n pouvant désigner un nombre de 0 à 1999 et de préférence de 49 à 149 et m pouvant désigner un nombre entier de 1 à 2000 et de préférence de 1 à 10;
   R' est un radical monavalent de formule CqE₂qL dans laquelle q est un nombre de 2 à 8 et L est choisi parmi les groupements :
   NR" - CH₂ - CH₂ - N(R")₂
   N(R")₂
   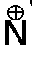 (R")₃A^{⊖}
   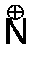 (R")H₂ A^{⊝}
   NR"CH₂ - CH₂- 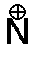 R"H₂ A^{⊝} dans lesquelles R" peut désigner hydrogène, phényle, benzyle, un radical hydrocarboné saturé monovalent et de préférence un radical alkyle ayant de 1 à 20 atomes de carbone et A⁹ représente un ion halogénure tel que fluorure , chlorure, bromure ou iodure.

Un produit particulièrement intéressant entrant dans cette définition est le polymère dénommé "triméthylsilyla- modiméthicone" répondant à la formule : 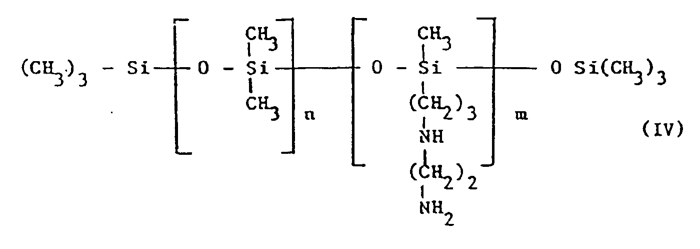 dans laquelle n et m ont les significations données ci-dessus (cf. formule III). De tels poylymères sont décrits dans la demande de brevet EP-A-95238.

(c) les polymères cationiques siliconés répondant à la formule : 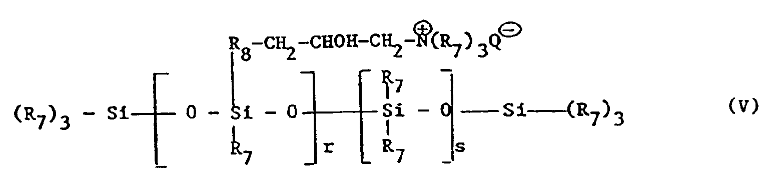 dans laquelle
R₇ désigne un radical hydrocarboné monovalent ayant de 1 à 18 atomes de carbore et en particulier un radical alkyle ou alcényle et de préférence méthyle;
R₈ désigne un radical hydrocarboné divalent, de préférence un radical alkylène en C₁-C₁₈ ou un radical alky- lènoxy divalent en C₁-C₁₈ et de préférence en C₁-C₈;
Q- est un ion halogénure, de préférence chlorure ;
r représente une valeur statistique moyenne de 2 à 20 et de préférence de 2 à 8;
s représente une valeur statistique moyenne de 20 à 200 et de préférence de 20 à 50.

De tels polymères sont décrits plus particulièrement dans le brevet US-4 185 087.

Un polymère entrant dans cette classe est le polymère vendu par la Société UNION CARBIDE sous la dénomination "UCAR SILICONE ALE 56".

Lorsque ces polymères siliconés sont mis en oeuvre, une forme de réalisation particulièrement intéressante est leur utilisation conjointe avec des agents de surface cationiques, éventuellement des agents de surface non ioniques. On peut utiliser par exemple dans les compositions conformes à l'invention le produit commercial vendu sous la dénomination "EMULSION CATIONIQUE DC 929" par la Société DOW CORNING qui comprend, outre l'amodiméthicone, un agent de surface cationique répondant à la formule : 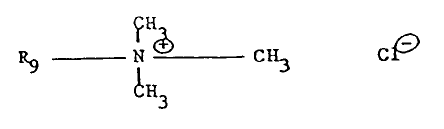 dans laquelle Rg désigne un mélange de radicaux alcényle et/ou alcoyle ayant de 14 à 22 atomes de carbone,dérivés des acides gras du suif, et un agent de surface non ionique de formule : 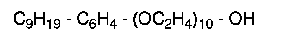 connu sous la dénomination "NONOXYNOL 10"®.

Une autre composition utilisable dans cette forme de réalisation de l'invention-est la composition contenant le produit vendu sous la dénomination "DOW CORNING Q2 7224"@ par la Société DOW CORNING comportant en association le triméthylsilylamodimethicone de formule (IV) un agent de surface non ionique de formule : 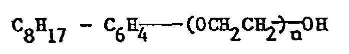 où n = 40 dénommé encore octoxynol-40, un autre agent de surface non ionique de formule : 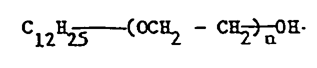 où n = 6 encore dénommé isolaureth-6,
et du glycol.

Les polymères du type polyamine, polyaminoamide, polyammonium quaternaire, utilisables conformément à la présente invention sont décrits en particulier dans les brevets français de la demanderesse n° 82 07 996 ou 84 04 475. Parmi ces polymères on peut citer :
(1) les copolymères vinyl-pyrrolidone-acrylate ou méthacrylate de dialkylaminoalkyle quaternisés ou non tels que les produits vendus sous la dénomination "GAFQUAT"@ par la Société GAF CORPORATION comme par exemple "GAFQUAT 734 ou 755"@ ou bien le produit dénommé "COPOLYMERE 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597 et en particulier les polymères commercialisés sous les dénominations "JR" (JR 400, JR 125, JR 30M)@ ou 'LR" (LR 400, LR 30M)® par la Société UNION CARBIDE CORPORATION. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire et décrits plus en détail dans le brevet américain 4 131 576 tels que les hydroxyalkyl celluloses comme les hydroxyméthyl-, hydroxyéthyl- ou hydroxypropyl cellulose greffées avec un sel de méthacryloyléthyl triméthylammonium, méthacrylamidopropyl triméthylammonium, diméthyl- diallylammonium.

Les produits commercialisés répondant à cette définition sont plus particulièrement les produits vendus sous la dénomination "CELQUAT L 200"@ et "CELQUAT H 100"@ par la Société NATIONAL STARCH.
(4) Les polysaccharides cationiques décrits plus particulièrement dans les brevets américains 3 589 578 et 4 031 307 et plus particulièrement le produit commercialisé sous la dénomination "JAGUAR C. 13 S"@ vendu par la Société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques ainsi que les produits d'oxydation et/ou de quaternisation de ces polymères. De tels polymères sont décrits dans les brevets français 2 162 025 et 2 280 361.
(6) Les polyaminopolyamides solubles dans l'eau préparés en particulier par polycondensation d'un composé acide avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alcoyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine. d'un diépoxyde ou d'un dérivé bis-insaturé; l'agent réticulant étant utilisé notamment dans des proportions allant de 0,025 à 0,35 mole par groupement amine du polyaminopolyamide.

Ces polyaminopolyamides peuvent être alcoylés ou s'ils comportent une ou plusieurs fonctions amines tertiaires, quaternisés. De tels polymères sont décrits en particulier dans les brevets français 2 252 840 et 2 368 508.
(7) Les dérivés de polyaminopolyamides résultant de la condensation de polyalcoylène polyamines avec des acides polycarboxyliques suivie d'une alcoylation par des agents bifonctionnels. On peut citer par exemple les polymères acide adipique-dialcoylaminohydroxyalcoyldialcoylene triamine dans lesquels le radical alcoyle comporte de 1 à 4 atomes de carbone et désigne de préférence méthyle, éthyle ou propyle. De tels polymères sont décrits dans le brevet français 1 583 363.

Parmi ces dérivés, on peut citer plus particulièrement les polymères acide adipique/diméthylamino hydroxy- propyl/diéthylènetriamine vendus sous les dénominations "CARTARETINE F, F₄ ou F8"® par la Société SANDOZ. (8) Les polymères obtenus par réaction d'une polyalkylène polyamine comportant deux groupements amine primaire et au moins un groupement amine secondaire avec un acide dicarboxylique choisi parmi l'acide diglycolique, et des acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminopolyamide en résultant étant amené à réagir avec l'épichlorhydrine dans un rapport molaire d'épichlorhydrine par rapport au groupement amine secondaire du polyaminopolyamide compris entre 0,5 : 1 et 1,8 : 1. De tels polymères sont décrits en particulier dans les brevets américains 3 227 615 et 2 961 347.

Des polymères de ce type sont en particulier commercialisés sous la dénomination "HERCOSETT 57"@ par la Société HERCULES INCORPORATED ou bien sous la dénomination de "PD 170"@ ou "DELSETTE 101"@ par la Société HERCULES dans le cas du copolymère d'acide adipique/époxypropyl/diéthylène-triamine.

9) Les cyclopolymères ayant un poids moléculaire de 20 000 à 3 000 000 tels que des homopolymères comportant comme constituant principal de la chaîne des motifs répondant aux formules (IX) ou (IX') 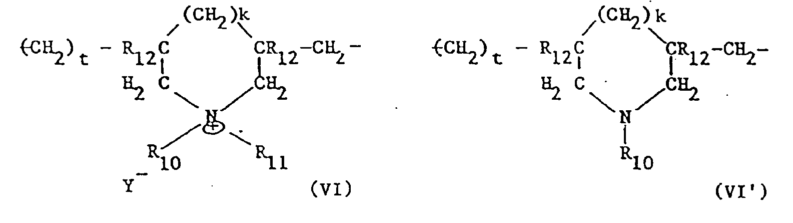 k et t sont égaux à 0 ou 1, et la somme k + t = 1 , R₁₂ désigne hydrogène ou méthyle, R₁ et R₁₁ désignent indépendamment l'un de l'autre, un groupement alcoyle ayant de 1 à 22 atomes de carbone, un groupement hydroxyalcoyle dans lequel le groupement alcoyle a de préférence 1 à 5 atomes de carbone, un groupement amidoalcoyle inférieur et où R₁ et R₁₁ peuvent désigner conjointement avec l'atome d'azote auquel ils sont rattachés des groupements hétérocycliques tels que pipéridinyle ou morpholinyle, ainsi que les copolymères comportant les motifs de formule (VI) ou (VI') et des motifs dérivés d'acrylamide ou de diacétone acrylamide, Y⁹ est un anion tel que bromure, chlorure, acétate, borate, citrate, tartrate, bisulfate, bisulfite, sulfate, phosphate. Parmi les polymères définis ci-dessus on peut citer plus particulièrement l'homopolymère de chlorure de diméthyl diallyl ammonium vendu sous la dénomination MERQUAT 100@ ayant un poids moléculaire inférieur à 100 000 et le copolymère de chlorure de diméthyl diallyl ammonium et d'acrylamide ayant un poids moléculaire supérieur à 500 000 et vendu sous la dénomination MERQUAT 550@ par la Société MERCK.

Ces polymères sont décrits plus particulièrement dans le brevet français 2 080 759 et son certificat d'addition n° 2 190 406.

10) Le polymère de polyammonium quaternaire contenant des motifs récurrents répondant à la formule : 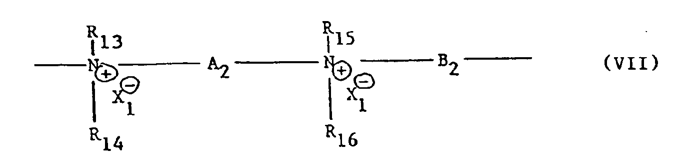 dans laquelle R₁₃ et R₁₄, R₁₅ et R₁₆ étant identiques ou différents, représentent des radicaux aliphatiques, alicy- cliques ou arylaliphatiques contenant de 1 à 20 atomes de carbone ou des radicaux hydroxyalkylaliphatiques inférieurs, ou bien R₁₃ et R₁₄ et R₁₅ et R₁₆ ensemble ou séparément, constituent avec les atomes d'azote auxquels ils sont rattachés, des hétérocycles contenant éventuellement un second hétéroatome autre que l'azote ou bien R₁₃, R₁₄, R₁₅ et R₁₆ représentent un radical alkyle en C₂-C₆ linéaire ou ramifié substitué par un groupement nitrile, ester, acyle, amide ou 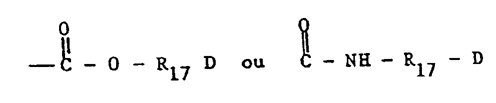 où R₁₇ est un alkylène et D un groupement ammonium quaternaire.
A₂ et B₂ représentent des groupements polyméthyléniques contenant de 2 à 20 atomes de carbone pouvant être linéaires ou ramifiés, saturés ou insaturés et pouvant contenir, liés à ou intercalés dans la chaîne principale, un ou plusieurs cycles aromatiques, ou un ou plusieurs atomes d'oxygène, de soufre ou des groupements SO, S0₂, disulfure, amino, alkylamino, hydroxyle, ammonium quaternaire, uréïdo, amide ou ester et
X⊖₁ désigne un anion dérivé d'un acide minéral ou organique.
A₂ et R₁₃ et R₁₅ peuvent former avec les deux atomes d'azote auxquels ils sont rattachés, un cycle pipérazi- nique; en outre si A₂ désigne un radical alkylène ou hydroxyalkylène linéaire ou ramifié, saturé ou insaturé, B₂ peut également désigner un groupement :
   - (CH₂)ₙ - CO - D - OC - (CH₂)ₙ-

dans lequel D désigne :
a) un reste de glycol de formule : O - Z - O -
   où Z désigne un radical hydrocarboné linéaire ou ramifié ou un groupement répondant à la formule : 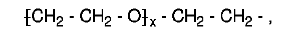 ou 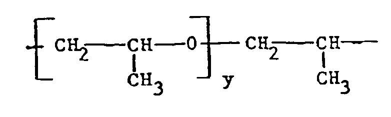 où x et y désignent un nombre entier de 1 à 4, représentant un degré de polymérisation défini et unique ou un nombre quelconque de 1 à 4 représentant un degré de polymérisation moyen;
b) un reste de diamine bis-secondaire tel qu'un dérivé de pipérazine,
c) un reste de diamine bis-primaire de formule : 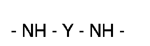 où Y désigne un radical hydrocarboné linéaire ou ramifié ou bien le radical bivalent 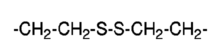
d) un groupement uréylène de formule : 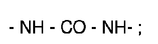 X⊖ est un anion tel que chlorure ou bromure.

Ces polymères ont une masse moléculaire généralement comprise entre 1000 et 100 000.

Des polymères de ce type sont décrits en particulier dans les brevets français 2 320 330, 2 270 846, 2 316 271, 2 336 434, et 2 413 907 et les brevets US-A- 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.

11) Les polymères de polyammonium quaternaires constitués de motifs de formule : 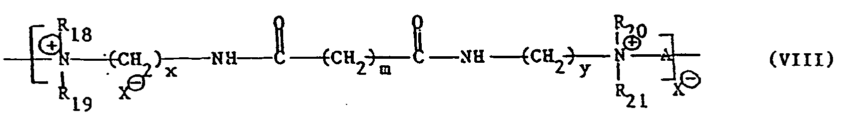 dans laquelle R₁₈, Rᵢg, R₂₀ et R₂₁, identiques ou différents représentent un atome d'hydrogène ou un radical méthyle, éthyle, propyle, β-hydroxyéthyle, β-hydroxypropyle ou -CH₂CH₂(OCH₂CH₂)ₚOH où p est égal à 0 ou un nombre entier compris entre 1 et 6, sous réserve que R₁₈, R₁₉, R₂₀ et R₂₁ ne représentent pas simultanément un atome d'hydrogène, x et y, identiques ou différents, sont des nombres entiers compris entre 1 et 6;
m est égal à 0 ou un nombre entier compris entre 1 et 34,
X désigne un atome d'halogène,
A désigne le reste d'un radical dihalogénure et représente de préférence

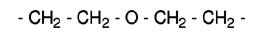

De tels composés sont décrits plus en détail dans la demande de brevet européen 122 324. 12) Les homopolymères ou copolymères dérivés d'acides acrylique ou méthacrylique et comportant des motifs : 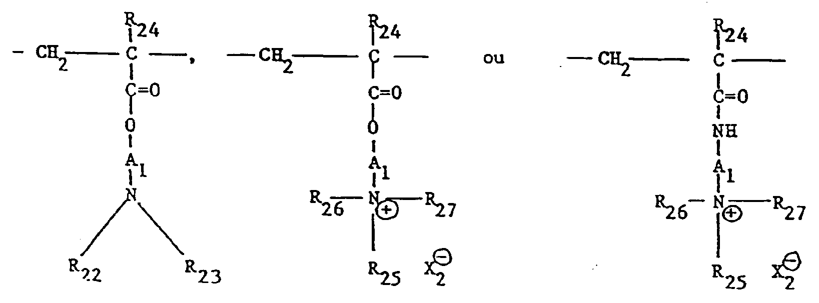 dans lesquels R₂₄ désigne H ou CH₃.
A₁ est un groupe alcoyle linéraire ou ramifié de 1 à 6 atomes de carbone ou un groupe hydroxyalcoyle de 1 à 4 atomes de carbone,
R₂₅, R₂₆, R₂₇ identiques ou différents représentent un groupe alcoyle ayant de 1 à 18 atomes de carbone ou un radical benzyle,
R et R₂₃ représentent hydrogène ou un groupe alcoyle ayant de 1 à 6 atomes de carbone,
X 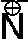 désigne un anion méthosulfate ou un halogénure tel que chlorure ou bromure.

Le ou les comonomères utilisables appartiennent à la famille des : acrylamide, méthacrylamide, diacétone acrylamide, acrylamide et méthacrylamide substitués à l'azote par des alcoyles inférieurs, des esters d'alcoyles des acides acrylique ou méthacrylique, la vinylpyrrolidone, des esters vinyliques. 13) Les polymères quaternaires de vinylpyrrolidone et de vinylimidazole tel que par exemple les produits commercialisés sous les dénominations LUVIQUAT FC 905, FC 550 et FC 3700 par la Société B.A.S.F

D'autres polymères cationiques utilisables conformément à l'invention sont des polyalkylènes imines en particulier des polyéthylèneimines, des polymères contenant des motifs vinylpyridine ou vinylpyridinium, des condensats de polyamines et d' épichlorhydrine, les polyuréylènes quaternaires et les dérivés de là chitine.

Parmi les polymères cationiques utilisables dans les compositions de l'invention, on citera notamment les polymères suivants :
le polymère comportant des motifs de formule : 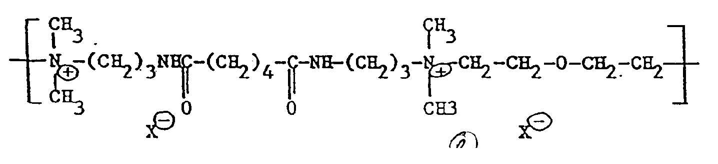 vendu sous la dénomination "MIRAPOL AD 1"® par la Société MIRANOL,
le polymère comportant les motifs de formule : 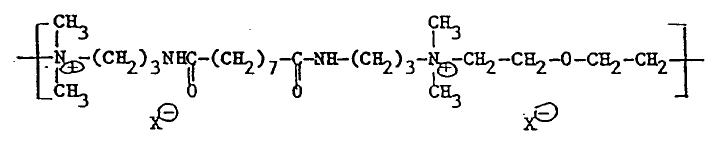 vendu sous la dénomination "MIRAPOL AZ1 "® par la Société MIRANOL,
le poly (chlorure de méthacrylamidopropyltriméthylammonium) vendu sous la dénomination "POLYMAPTAC"® par la Société TEXACO CHEMICALS;
un polymère quaternisé du type ionène décrit dans le brevet français de la demanderesse n° 2 270 846 et plus particulièrement ceux comportant les motifs : 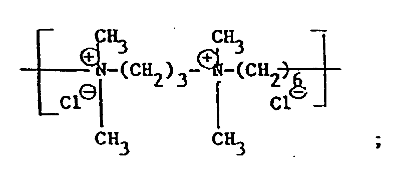
les cyclopolymères de diméthyldiallyl ammonium vendus sous les dénominations "MERQUAT 100"® et "MERQUAT 550"® par la Société MERCK;
les polymères quaternaires de vinylpyrrolidone et de vinylimidazole tels que ceux vendus sous les dénominations "LUVIQUAT FC 905, FC 550 et FC 370"@ par la Société B.A.S.F;
les copolymères de vinylpyrrolidone/acrylate ou méthacrylate de dialkylaminoalkyle quaternisés ou non tels que les produits vendus sous les dénominations "COPOLYMERE 937"@, "GAFQUAT 734 ou 755"® par la Société GAF;
les polymères d'éthers de cellulose quaternaires tels que ceux vendus sous les dénominations "JR" comme par exemple JR 125@, JR 400@, JR 30-M® et LR® tels que LR 400 et LR 30 par la Société UNION CARBIDE CORPORATION;
les dérivés de cellulose cationiques tels que les produits vendus sous les dénominations "CELQUAT L 200"® et CELQUAT H 100"® par la Société National Starch;
les polymères d'ammonium quaternaires du type décrit dans le brevet US 4 157 388 et plus particulièrement le polymère comportant des motifs de formule : 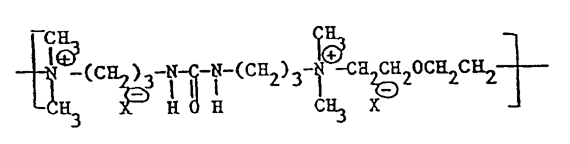 vendu sous la dénomination de "MIRAPOL A 15"® par la Société MIRANOL;
- le poly (chlorure de diméthyl butényl ammonium)- α,ω-bis-(chlorure de triéthanol ammonium) vendu sous la dénomination de "ONAMER M"@ par la Société ONYX INTERNATIONALE.
- les polymères aminés à squelette siliconé tels que l'amodimethicone contenu dans l'émulsion cationique DC929 commercialisée par DOW CORNING.

Les particules revêtues présentes dans la composition de l'invention comprennent de préférence des charges ou des pigments minéraux.

Les charges minérales, naturelles ou synthétiques sont par exemple choisies parmi : les carbonates de calcium, les silicates comme par exemple le silicate d'aluminium ou kaolin, les silicates de calcium, l'alumino-silicate de sodium, le silicate de magnésium ou talc, les alumino-silicates de potassium ou micas et l'alumino-silicate de magnésium hydraté ; les sulfates comme par exemple le sulfate de baryum, le sulfate de calcium ; les dioxydes de silicium précipités ou pyrogénés, ainsi que les hydrogels et aérogels de silice.

Les pigments sont choisis par exemple parmi les pigments blancs tels que le dioxyde de titane ou l'oxyde de zinc et les pigments colorés tels que : des oxydes de fer colorés (naturels ou synthétiques) de couleurs noire, rouge et jaune ; des oxydes de chrome verts hydratés ou non ; le bleu de Prusse ; les alumino-sulfosilicates de sodium et leurs différentes variantes connues sous le nom de pigments "ultramarines" ; l'aluminate de cobalt ou bleu de cobalt et le violet de manganèse.

Les pigments peuvent être encore :
- soit des pigments nacrés tels que les mica-titanes (mica revêtu de particules de dioxyde de titane) et l'oxychlorure de bismuth ;
- soit les pigments micronisés d'oxydes métalliques choisis parmi les oxydes de titane, de zinc, de cérium, de zirconium ou leurs mélanges.

Les pigments de départ destinés à être revêtus par un polymère cationique, conformément à l'invention, peuvent être des pigments ayant subi un ou plusieurs traitements de surface de nature chimique, électronique, mécanochimique et/ou mécanique avec des composés tels que décrits par exemple dans Cosmetics & Toiletries, Février 1990. Vol. 105, p. 53-64, notamment avec des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), des polyéthylènes, des silicones, des protéines (collagène, élastine), des alcanolami- nes, des oxydes de silicium, des oxydes métalliques ou de l'hexamétaphosphate de sodium.

Dans les compositions de l'invention, les proportions des particules revêtues, dispersées dans le liant, dépendent du type de compositions ; il s'agit des proportions usuelles pour le type de composition considéré.

Pour enrober les particules, on peut utiliser une méthode connue, par exemple l'une des méthodes suivantes :
1) On prépare une solution du polymère dans un de ses bons solvants. On disperse dans cette solution la poudre à enrober sous agitation vigoureuse et on ajoute un mauvais solvant du polymère sans aller jusqu'à la précipitation du polymère dans la solution, mais jusqu'au premier trouble. On laisse alors la suspension sous agitation vigoureuse, par exemple pendant 4 heures. On laisse décanter la suspension, on rince avec un non-solvant du polymère et on sèche, par exemple à 80°C sous pression réduite.
2) On prépare une solution du polymère dans laquelle on disperse la poudre à enrober. On laisse le système sous agitation vigoureuse et on ajoute lentement un précipitant du polymère de façon à faire précipiter doucement le polymère à la surface de la poudre. On laisse décanter, on rince avec un non-solvant du polymère et on sèche la poudre.
3) On prépare une solution, avec un bon solvant du polymère, et on y disperse la poudre à enrober. On choisit un mauvais solvant du polymère dont le point d'ébullition est supérieur à celui du bon solvant et on réalise une évaporation lente du système. On aboutit à la formation d'un coacervat qui enrobe progressivement la poudre, puis on sèche la poudre.
4) On utilise la technique dite du lit d'air fluidisé : on pulvérise, à chaud, une solution diluée du polymère dans un cyclone dans lequel la poudre est maintenue en sustentation.
5) On prépare une solution du polymère dans laquelle on disperse la poudre à enrober. On laisse le système sous agitation vigoureuse et on évapore lentement le solvant de façon à faire précipiter doucement le polymère à la surface de la poudre. On laisse décanter, on rince avec un non-solvant du polymère, et on sèche la poudre.
6) Procédé par atomisation :
   On prépare une solution du polymère dans laquelle on disperse la poudre à enrober. On aspire la solution par une pompe et on la fait passer dans une gaine chauffante où le solvant est évaporé. On récupère la poudre enrobée à l'aide d'un aspirateur dans un cyclone.
7) Procédé par lyophilisation :
   On prépare une solution du polymère dans laquelle on disperse la poudre à enrober. On congèle le mélange et on le met sous vide. On récupère alors la poudre enrobée.

Certaines méthodes particulières d'enrobage de pigments à l'aide de polymères sont décrites dans les documents FR-A-2234359 et EP-A-249685.

Les compositions de l'invention peuvent être des compositions anhydres. Les compositions anhydres se présentent notamment sous la forme d'une poudre compactée, d'une poudre coulée, d'un rouge à lèvres ou d'un vernis à ongles.

Les compositions de l'invention peuvent également se présenter sous la forme d'émulsions du type eau-dans- l'huile ou huile-dans-l'eau, et être employées comme fond de teint ou mascara par exemple.

Ces compositions sont préparées selon les méthodes usuelles.

Dans les compositions de maquillage, le liant est un liant gras usuel constitué d'une huile, d'un mélange d'huiles ou d'un mélange d'huile et de cire(s).

Dans les rouges à lèvres, le liant est également un liant gras généralement constitué par un mélange de cires de haut point de fusion (naturelles ou synthétiques), d'huiles (synthétiques, minérales ou végétales) et de cires à bas point de fusion (naturelles ou synthétiques).

Dans les vernis à ongles, le liant est constitué par la solution du polymère filmogène et du plastifiant dans le solvant organique choisi.

Dans les émulsions, le liant est notamment un liant gras usuel constitué par exemple d'une huile ou d'un mélange d'huiles.

Lorsque la composition comporte un pigment micronisé d'oxydes métalliques choisis parmi les oxydes de titane, de zinc, de cérium, de zirconium ou leurs mélanges, elle peut constituer une composition protectrice de l'épiderme humain contre les rayons ultra violets.

L'invention concerne également l'utilisation, dans la préparation d'une composition cosmétique, contenant une dispersion de particules solides dans un liant, de particules dont la surface est revêtue avec au moins un polymère cationique. Dans cette utilisation, la composition, et notamment les particules, le polymère cationique, ainsi que le liant, sont en particulier tels que définis ci-dessus.

L'invention concerne en outre un procédé de traitement cosmétique, caractérisé par le fait que l'on applique sur la peau ou les phanères une composition cosmétique telle que définie précédemment.

L'application des compositions de l'invention est effectuée de manière et en quantité usuelles.

Les exemples suivants illustrent l'invention.

### EXEMPLE DE PREPARATION 1

On introduit dans 100ml d'eau 30g d'un copolymère de vinylpyrrolidone et de diméthyl-amino-éthyl-méthacrylate quaternisé au sulfate de diéthyle, contenant 80 % en poids de motifs vinylpyrrolidone (GAFQUAT 755N@ de la Société GAF).

Parallèlement on prépare une dispersion de 75g de talc dans 230g d'eau. Quand on obtient une dispersion correcte, on verse celle-ci dans la solution de copolymère et on laisse sous agitation pendant 4 heures. On ajoute alors 710 ml d'acétone et on laisse à nouveau sous agitation pendant 4 heures. On laisse décanter la préparation, le talc est récupéré, rincé à l'acétonitrile, séché, broyé et tamisé. L'analyse élémentaire du résidu organique, montre que l'on a obtenu une poudre revêtue contenant 2,41%, en poids, du polymère cationique.

De façon analogue, on a revêtu de l'oxyde de chrome et des mica-titanes :
- oxyde de chrome commercialisé par Whittaker sous la dénomination Ultra Green BC 7109® ;
- mica-titane 31/69 commercialisé par Mearl sous la dénomination Flamenco Satina@
- mica-titane 83/17 commercialisé par Mearl sous la dénomination Timaca Sparkle®.

### EXEMPLE DE PREPARATION 2

On dissout 150,9 g d'un polymère de chlorure de méthacrylamidopropyltriméthylammonium dans 1300 ml d'eau. Parallèlement on prépare une dispersion de 500 g de talc dans 1500 ml d'eau. On ajoute la dispersion dans la solution de polymère, et on laisse sous agitation pendant 4 heures. La poudre est récupérée par centrifugation, et le culot est rincé avec 500 ml d'acétonitrile, puis séché, broyé et tamise.

Le polymère cationique utilisé est le Polymaptac®, commercialisé par la Société TEXACO CHEMICALS. Le talc revêtu contient 2 % du polymère cationique.

De façon analogue, on a revêtu de l'oxyde de chrome et des mica-titanes.

### EXEMPLE DE PREPARATION 3

On opère de façon analogue à celle décrite à l'exemple 1, avec 82,8 g de polymère cationique dans 1500 ml d'eau. La poudre décantée est rincée avec 2600 ml d'isopropanol.

Le polymère cationique utilisé est le MEXOMERE PO® commercialisé par la Société CHIMEX. IL s'agit d'un polymère résultant de la condensation de la N, N'-tétraméthylpropylènediamine sur le 1,6-dichlorohexane.

La proportion de polymère cationique dans le talc revêtu est de 5 %.

De façon analogue, on a revêtu de l'oxyde de chrome et des mica-titanes.

### EXEMPLE DE PREPARATION 4

On utilise comme produit de départ 142,3g d'un polymère cationique siliconé porteur d'une chaîne latérale terminée par une fonction amine primaire, (amodiméthicone contenue dans l'émulsion DC 929^{(R)} de Dow Corning). On dilue cette émulsion avec 1400 ml d'eau.

Parallèlement on prépare une dispersion de talc en introduisant 500 g de talc dans 1500 ml d'eau.

On ajoute la dispersion ainsi réalisée à l'émulsion de polymère siliconé et on laisse sous agitation pendant 4 heures. On distille ensuite l'eau, de façon à réaliser la coalescence de l'émulsion, et l'adsorption du polymère sur le talc.

Le produit est rincé à l'eau, séché à l'étuve sous vide, broyé et tamisé. Le talc revêtu contient 3 % du polymère cationique.

### EXEMPLE DE PREPARATION 5

On introduit dans 1400 ml d'eau, 250 g d'une poly(éthylène-imine), polymère cationique qui contient des fonctions amines secondaires et primaires.

Parallèlement on prépare une dispersion de talc en introduisant 500 g de talc dans 1600 ml d'eau.

On introduit la dispersion de talc dans la solution de polymère, et on laisse sous agitation pendant 4 heures.

On récupère le talc revêtu qui est rincé à l'acétonitrile, puis séché en étuve, broyé et tamisé. Il contient 0,4 % du polymère cationique.

La poly(éthylène-imine) utilisée est commercialisée sous la dénomination POLYMINE SK® par la Société BASF

### EXEMPLE DE PREPARATION 6 : Enrobage par atomisation

On dissout dans 400 ml d'eau, 1,66 g de polymère MEXOMERE PO® en solution à 60 % en poids dans l'eau, et on y disperse 100 g de dioxyde de titane micronisé. Il faut que le mélange soit suffisamment fluide (on rajoute de l'eau si nécessaire). On laisse le mélange sous agitation pendant 1 heure et on procède ensuite à l'atomisation à l'aide de l'appareil BUCHI 190 - Mini Spray Dryer.

Conditions opératoires :
- Débit : 600-700
- Température entrée : 130° C (graduation 10)
- Température sortie : 60 ⁰ C
- Aspirateur : 20
- Pompe : 10

Durant toute l'atomisation, le mélange reste sous agitation magnétique.

On récupère un produit très fin au toucher, sans besoin de le tamiser ni de le sécher. Le dioxyde de titane revêtu contient 1 % du polymère cationique.

### EXEMPLE DE PREPARATION 7 : Enrobage par lyophilisation

On dissout dans 50 ml d'eau, 0,606 g de polymère POLYMAPTAC® en solution à 33 % en poids dans l'eau, et on incorpore, sous agitation magnétique 10 g de talc. On laisse sous agitation magnétique pendant 5 heures.

On verse le mélange dans un récipient de 600 ml, on le congèle à l'aide d'azote liquide puis on le met sur le lyophi- lisateur modulaire VIRTIS® Modèle 10 020 pendant au moins 18 heures.

On récupère un produit assez pulvérulent que l'on tainise sur 100 µm. Le talc revêtu contient 2 % du polymère cationique.

### EXEMPLE DE PREPARATION 8 :

On opère de façon analogue à celle décrite à l'exemple 7, avec 0,893 g de protéine de blé quaternisée vendu sous la dénomination HYDROTRITICUM WQ@ par la Société CRODA (en solution à 28 % en poids dans l'eau) dans 50 ml d'eau et 10 g de talc.

On récupère un produit assez pulvérulent que l'on tamise sur 100 pm.

Le talc revêtu contient 2,5 % du polymère cationique.

### EXEMPLES DE COMPOSITIONS

### EXEMPLE A : Formulation d'un fard à paupières

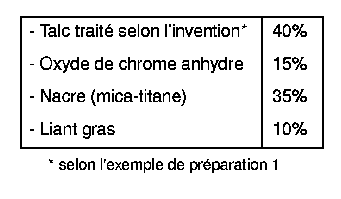

On réalise cette préparation cosmétique selon un mode opératoire classique de formulation en utilisant un agitateur-démotteur du type Baker-Perkin. La nacre est introduite à la fin du mélange des poudres. Quand le mélange est homogène on ajoute le liant gras sous agitation réduite. Ce liant est constitué de :

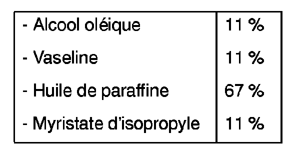

Sur les cosmétiques ainsi préparés, on a effectué un test d'évaluation sensorielle. Pour ce faire on a constitué un groupe de 25 personnes testrices qui ont évalué ces cosmétique selon un critère d'appréciation qui est la faculté à faire des stries. Le test consiste à appliquer la formule testée sur les paupières. Le résultat est jugé immédiatement après application et 4 heures après. Lorsqu'une formule présente une bonne adhérence sur la peau le maquillage reste uniforme plusieurs heures après l'application. Lorsque l'adhérence est mauvaise, il y a apparition de stries qui séparent les zones où le maquillage adhère de celles où le maquillage s'est détaché de la peau. Les formules utilisant des talcs revêtus selon l'invention ont été jugées toujours très supérieures à celles utilisant des talcs non revêtus.

### EXEMPLE B : Fards à paupières compactés -Etude des propriétés mécaniques

### FORMULATION

On a préparé des fards à paupières compactés (ci-après : "compacts") selon les formulations indiquées dans le tableau 1 ci-après.

Le liant est celui décrit à l'exemple A ci-dessus.

### TEST DE CHUTE

On réalise 9 chutes successives du compact à 20 cm de hauteur. On note la perte de poids du compact après les 9 chutes.

### TEST DE DURETE

La dureté est mesurée avec un duromètre de la marque ZWICK.

Elle est exprimée en degrés Shore.

De façon générale, on cherche à trouver un compromis qui permette d'obtenir un compact qui puisse se déliter (pour prélever du produit et l'appliquer) mais qui soit suffisament dur pour présenter une bonne solidité et être manipulable sans précautions particulières et sans perte de produit.

La dureté est fonction de la pression de compactage qui permet d'obtenir des produits qui donnent une perte minimum dans le test de chute.

Les résultats sont résumés dans le Tableau 1 suivant.

Pressions de compactage :
- 60 b (60 bars) : 6.10⁶Pa
- 40 b (40 bars) : 4.10⁶Pa
- 30 b (30 bars) : 3.10⁶Pa

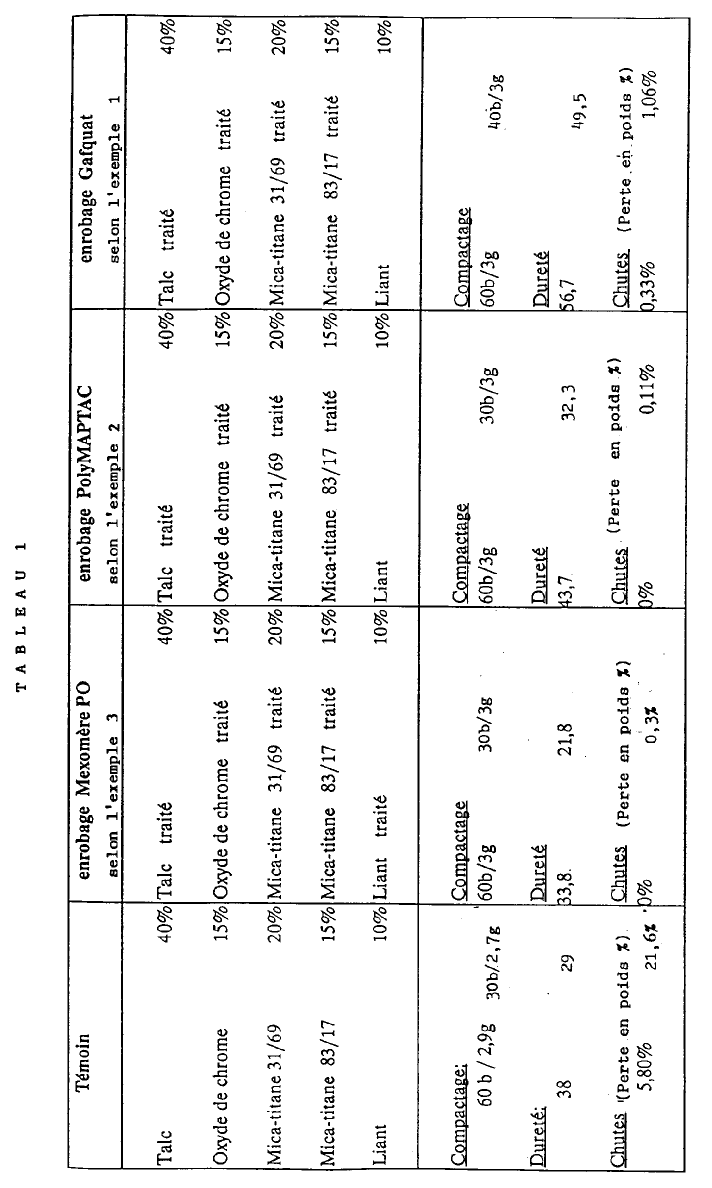

### EXEMPLE C:Fond de teint

On prépare, sous la forme d'une émulsion eau-dans-l'huile, un fond de teint ayant la formulation suivante :

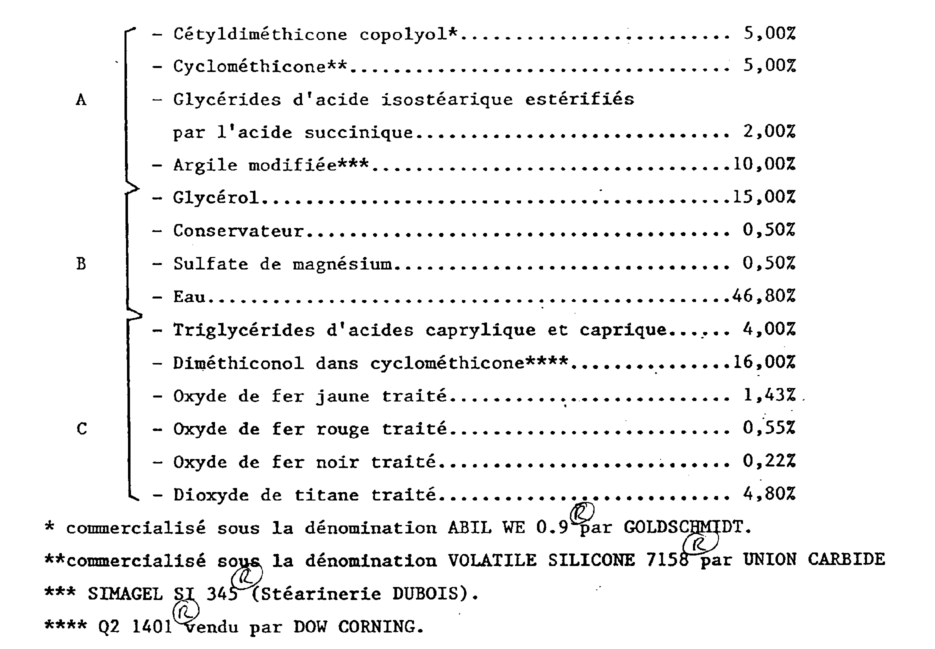

Pour préparer ce fond de teint, on opère de la façon suivante :
A l'ensemble A on ajoute à 70°C et sous agitation l'ensemble B. Une fois l'émulsion formée, on ajoute le mélange C.

Les pigments traités étaient revêtus par le GAFQUAT 755 N, selon le procédé de l'exemple 1.

### EXEMPLE D: Fond de teint

On prépare, sous la forme d'une émulsion huile-dans-l'eau, un fond de teint ayant la formulation suivante :

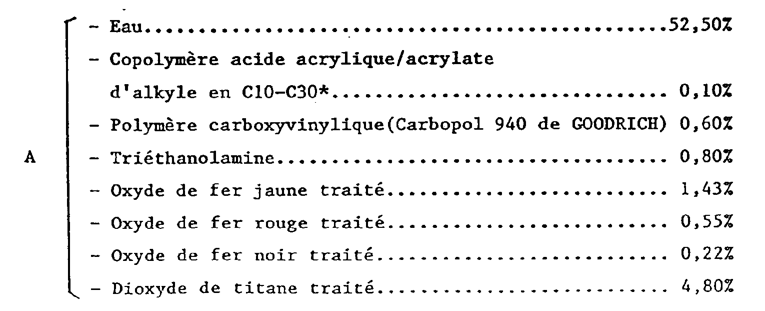

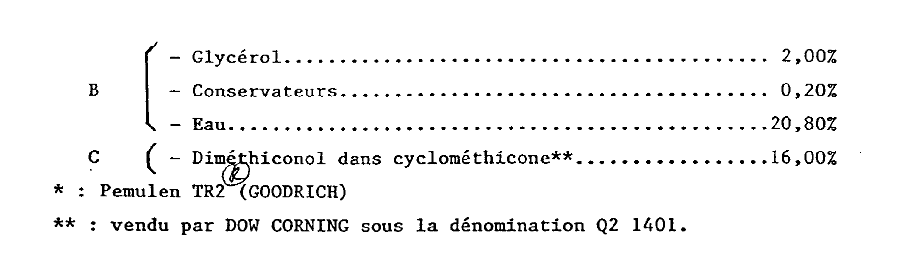

Pour préparer ce fond de teint, on opère de la façon suivante :
A l'aide d'un disperseur, on prépare à 70°C le mélange A. On ajoute, toujours à chaud, l'ensemble B. On prépare l'émulsion en ajoutant l'ensemble C au mélange A + B, en maintenant l'agitation jusqu'au retour à la température ambiante.

Les pigments traités sont des pigments qui ont été soumis à un traitement de revêtement comme décrit à l'exemple 1.

### EXEMPLE E : Vernis à ongle

Formulation :

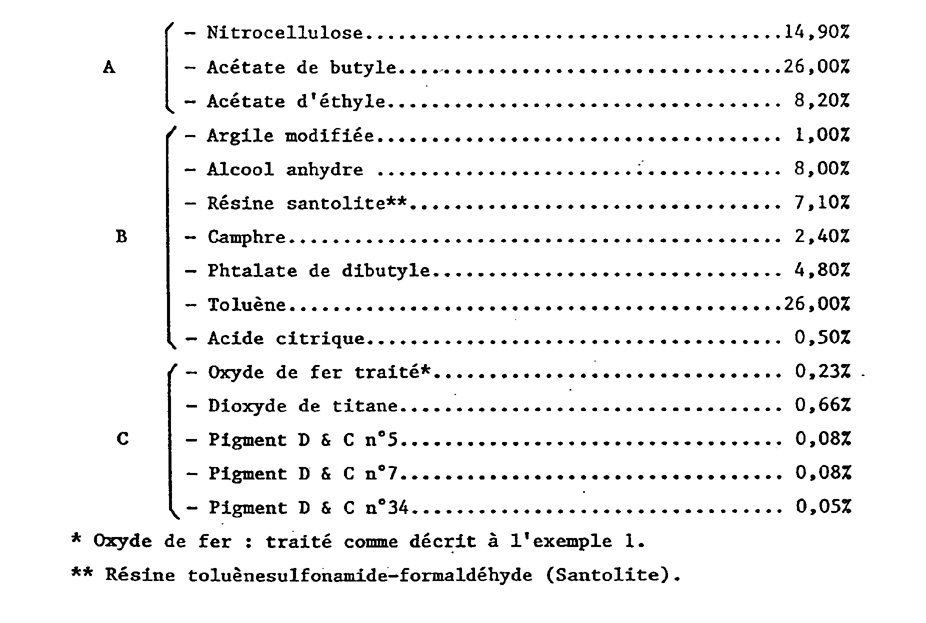

Pour préparer ce vernis à ongles, on opère constamment à température ambiante et sous agitation. On prépare séparément les mélanges A et B. On ajoute A dans B, puis on ajoute l'ensemble C constitué par les pigments.

Dans les exemples F, G, H qui suivent, les fards à paupières sont préparés de façon analogue à l'exemple A en introduisant les oxydes de fer à la fin du mélange des poudres.

### EXEMPLE : Fard à paupières

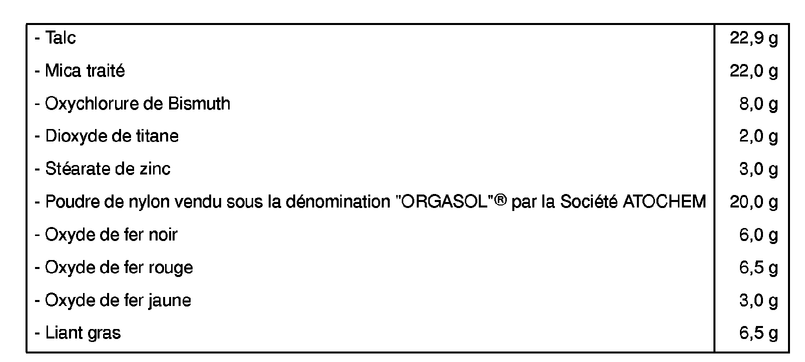

Le mica est revêtu par le GAFQUAT 755 N®, selon le procédé de l'exemple 1.

Le liant est celui décrit à l'exemple A ci-dessus.

### EXEMPLE G : Fard à paupières

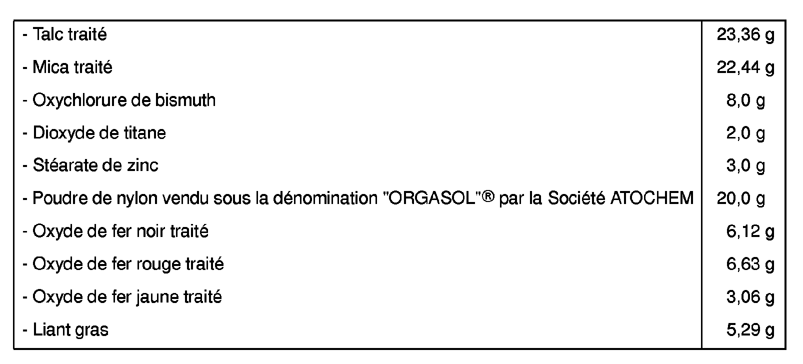

Les pigments traités sont revêtus par le POLYMAPTAC® selon le procédé de l'exemple 7.

Le liant est celui décrit à l'exemple A ci-dessus.

### EXEMPLE H : Fard à paupières

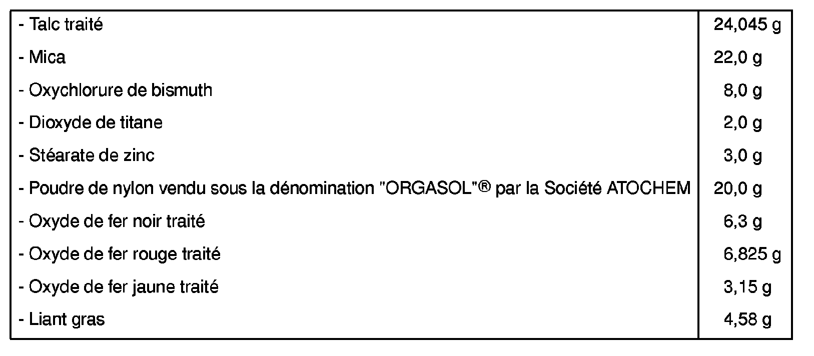

Les pigments traités sont revêtus par le MEXOMERE PO®, selon le procédé de l'exemple 6.

Le liant est celui décrit à l'exemple A ci-dessus.

Un test d'évaluation sensorielle a été effectué, de façon analogue à celui décrit dans l'exemple A, auprès de 16 personnes, avec les exemples F, G et H qui ont été comparés à une formule selon l'exemple F dans laquelle les pigments n'étaient pas revêtus. Les formules utilisant des pigments revêtus selon l'invention ont été jugées toujours supérieures à celles utilisant des pigments non revêtus.

### EXEMPLE 1 : Fard coulé

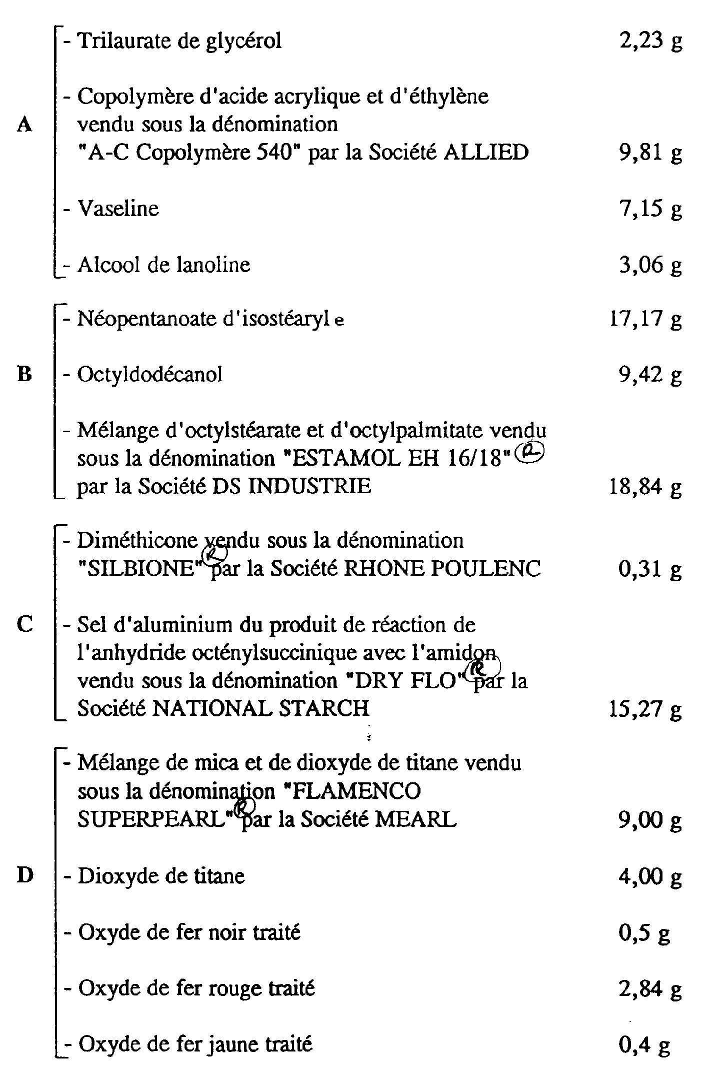

Les oxydes de fer sont revêtus par le GAFQUAT 755 N@, selon le procédé de l'exemple 1.

On broie le mélange D dans le mélange B. On ajoute le mélange A.

On chauffe à 80 - 90° C tout en agitant pour homogénéiser. On ajoute C.

On coule à chaud dans une coupelle.

### EXEMPLE J : Mascara

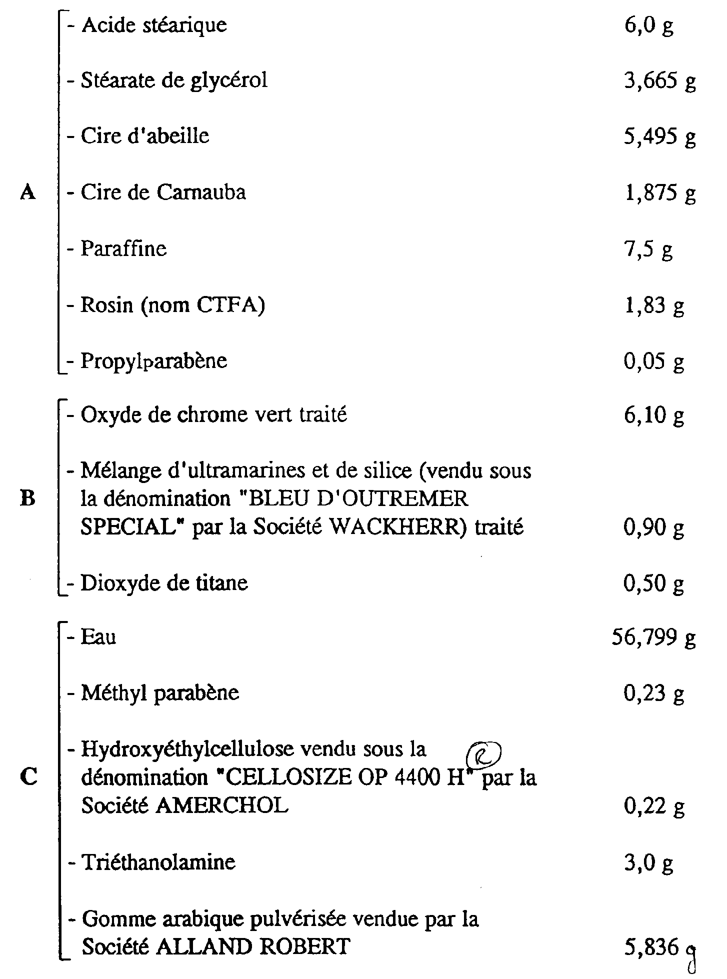

Les pigments traités sont revêtus par le POLYMAPTAC, selon le procédé de l'exemple 7.

On chauffe le mélange A à 90° C.

On y ajoute B puis on ajoute la phase aqueuse C chauffée à 70 - 80° C.

On agite 10 à 15 minutes pour obtenir l'émulsion et on laisse refroidir.

On obtient ainsi un mascara onctueux, présentant une dispersion fine des pigments.

### EXEMPLE K : Composition protectrice de l'épiderme humain

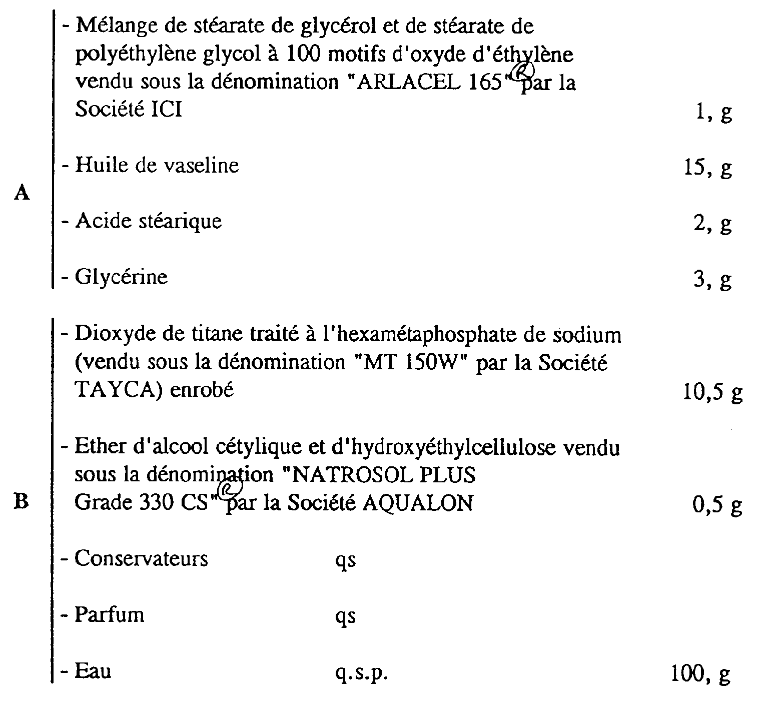

Le dioxyde de titane est revêtu à 5 % par le GAFQUAT 755 N@, selon le procédé de l'exemple 1.

On chauffe le mélange A à 80° C et on ajoute le mélange B à 80° C. On laisse refroidir sous agitation.

On obtient ainsi une émulsion Eau dans Huile pour la protection de l'épiderme humain contre le rayonnement ultraviolet.

## Revendications

1. Composition cosmétique pour la peau ou les phanères contenant une dispersion de particules solides dans un liant, caractérisée par le fait qu'au moins une partie desdites particules sont introduites dans ladite composition sous la forme de particules dont la surface est revêtue d'au moins un polymère cationique, ledit polymère cationique contenant des motifs comportant, soit dans la chaîne polymère, soit sur des substituants latéraux, des groupements amine primaire, amine secondaire, amine tertiaire et/ou ammonium quaternaire, et par le fait que ledit liant est un liant gras.

2. Composition anhydre de vernis à ongle contenant une dispersion de particules solides dans un liant, caractérisée par le fait qu'au moins une partie desdites particules sont introduites dans ladite composition sous la forme de particules dont la surface est revêtue d'au moins un polymère cationique, ledit polymère cationique contenant des motifs comportant, soit dans la chaîne polymère, soit sur des substituants latéraux, des groupements amine primaire, amine secondaire, amine tertiaire et/ou ammonium quaternaire, et par le fait que ledit liant est constitué par une solution d'un polymère filmogène et d'un plastifiant dans un solvant organique approprié.

3. Composition cosmétique pour la peau ou les phanères contenant une dispersion de particules solides dans un liant, caractérisée par le fait qu'au moins une partie desdites particules sont introduites dans ladite composition sous la forme de particules dont la surface est revêtue d'au moins un polymère cationique, ledit polymère cationique contenant des motifs comportant, soit dans la chaîne, soit sur des subsituants latéraux, des groupements ammonium quaternaire.

4. Composition selon l'une quelconque des revendications précédentes, caractérisée par le fait que lesdites particules revêtues comprennent des charges minérales ou des pigments minéraux.

5. Composition selon la revendication précédente, caractérisée par le fait que lesdites particules revêtues comprennent des charges choisies parmi : les carbonates de calcium, les silicates comme par exemple le silicate d'aluminium ou kaolin, les silicates de calcium, l'alumino-silicate de sodium, le silicate de magnésium ou talc, les alumino- silicates de potassium ou micas et l'alumino-silicate de magnésium hydraté ; les sulfates comme par exemple le sulfate de baryum, le sulfate de calcium ; les dioxydes de silicium précipités ou pyrogénés, ainsi que les hydrogels et aérogels de silice.

6. Composition selon la revendication 4, caractérisée par le fait que lesdites particules revêtues comprennent des pigments choisis parmi :
les pigments blancs tels que le dioxyde de titane ou l'oxyde de zinc et les pigments colorés tels que : des oxydes de fer colorés (naturels ou synthétiques) de couleurs noire, rouge et jaune ; des oxydes de chrome verts hydratés ou non ; le bleu de Prusse ; les alumino-sulfosilicates de sodium et les pigments ultramarines; l'aluminate de cobalt ou bleu de cobalt et le violet de manganèse.

7. Composition selon la revendication 4, caractérisée par le fait que lesdites particules revêtues comprennent des pigments nacrés tels que les mica-titanes et l'oxychlorure de bismuth.

8. Composition selon la revendication 4, caractérisée par le fait que lesdites particules revêtues comprennent des pigments de zinc, de cérium, de zirconium ou leurs mélanges.

9. Composition selon l'une quelconque des revendications précédentes, caractérisée par le fait que la proportion pondérale de polymères cationiques, dans les particules revêtues, est d'au moins 0,1 % en poids, par rapport au poids total des particules revêtues.

10. Composition selon la revendication précédente, caractérisée par le fait que ladite proportion est inférieure à 10 % et de préférence inférieure à 8 %.

11. Composition selon l'une quelconque des revendications précédentes, caractérisée par le fait que lesdites particules revêtues sont revêtues uniquement avec un ou plusieurs polymères cationiques.

12. Composition selon l'une quelconque des revendications précédentes, caractérisée par le fait que ledit polymère cationique contient au moins 10 % en poids desdits motifs comportant des groupements amine primaire, amine secondaire, amine tertiaire et/ou ammonium quaternaire.

13. Composition selon l'une quelconque des revendications précédentes, caractérisée par le fait que ledit polymère cationique contient des groupements amine ou ammonium quaternaire qui font partie de la chaîne polymère.

14. Composition selon la revendication 13, caractérisée par le fait que lesdits groupements ont un taux de quaternisation, par gramme de polymère, au moins égal à 0,05 méq cationique.

15. Composition selon l'une quelconque des revendications 1 à 12, caractérisée par le fait que lesdits groupements amine ou ammonium quaternaire sont portés par un substituant latéral.

16. Composition selon la revendication précédente, caractérisée par le fait que la chaîne polymère dudit polymère cationique est une chaîne acrylique, vinylique, siliconée, fluorée ou saccharidique.

17. Composition selon l'une quelconque des revendications précédentes, caractérisée par le fait qu'elle se présente sous la forme d'une poudre compactée, d'une poudre coulée, d'un rouge à lèvres, d'un vernis à ongles ou d'une émulsion.

18. Composition selon l'une quelconque des revendications précédentes, caractérisée par le fait qu'elle constitue une composition anhydre.

19. Procédé de traitement cosmétique destiné au maquillage de la peau ou des phanères, caractérisé par le fait que l'on applique sur la peau ou les phanères une composition telle que définie dans la revendication 1 ou dans la revendication 1 et l'une quelconque des revendications 3 à 18.

20. Procédé de traitement cosmétique, caractérisé par le fait que l'on applique sur les ongles une composition telle que définie dans la revendication 2 ou dans la revendication 2 et l'une quelconque des revendications 4 à 18.

## Claims

1. Cosmetic composition for the skin or superficial body growths, containing a dispersion of solid particles in a binder, characterized by the fact that at least a portion of the said particles are introduced into the said composition in the form of particles whose surface is coated with at least one cationic polymer, the said cationic polymer containing units including primary amine, secondary amine, tertiary amine and/or quaternary ammonium groups either in the polymer chain, or in side substituents, and by the fact that the said binder is a fatty binder.

2. Anhydrous nail varnish composition containing a dispersion of solid particles in a binder, characterized by the fact that at least a portion of the said particles are introduced into the said composition in the form of particles whose surface is coated with at least one cationic polymer, the said cationic polymer containing units including primary amine, secondary amine, tertiary amine and/or quaternary ammonium groups either in the polymer chain, or in side substituents, and by the fact that the said binder consists of a solution of a film-forming polymer and of a plasticizer in a suitable organic solvent.

3. Cosmetic composition for the skin or superficial body growths, containing a dispersion of solid particles in a binder, characterized by the fact that at least a portion of the said particles are introduced into the said composition in the form of particles whose surface is coated with at least one cationic polymer, the said cationic polymer containing units including quaternary ammomium groups either in the chain or in side substituents.

4. Composition according to any one of the preceding claims, characterized by the fact that the said coated particles comprise inorganic fillers or inorganic pigments.

5. Composition according to the preceding claim, characterized by the fact that the said coated particles comprise fillers chosen from: calcium carbonates, silicates such as for example aluminium silicate or kaolin, calcium silicates, sodium aluminosilicate, magnesium silicate or talc, potassium aluminosilicates or micas and hydrated magnesium aluminosilicate; sulphates such as for example barium sulphate, calcium sulphate; precipitated or pyrogenic silicon dioxides, as well as silica hydrogels and aerogels.

6. Composition according to Claim 4, characterized by the fact that the said coated particles comprise pigments chosen from: white pigments such as titanium dioxide or zinc oxide and coloured pigments such as:
coloured iron oxides (natural or synthetic) black, red and yellow in colour; hydrated or non-hydrated green chromium oxides; Prussian blue; sodium aluminosulphosilicates and ultramarine pigments; cobalt aluminate or cobalt blue and manganese violet.

7. Composition according to Claim 4, characterized by the fact that the said coated particles comprise pearly pigments such as mica-titaniums and bismuth oxychloride.

8. Composition according to Claim 4, characterized by the fact that the said coated particles comprise zinc, cerium, or zirconium pigments or mixtures thereof.

9. Composition according to any one of the preceding claims, characterized by the fact that the proportion by weight of cationic polymers, in the coated particles, is at least 0.1% by weight relative to the total weight of the coated particles.

10. Composition according to the preceding claim, characterized by the fact that the said proportion is less than 10% and preferably less than 8%.

11. Composition according to any one of the preceding claims, characterized by the fact that the said coated particles are coated solely with one or more cationic polymers.

12. Composition according to any one of the preceding claims, characterized by the fact that the said cationic polymer contains at least 10% by weight of the said units including primary amine, secondary amine, tertiary amine and/or quaternary ammonium groups.

13. Composition according to any one of the preceding claims, characterized by the fact that the said cationic polymer contains amine or quaternary ammonium groups which form part of the polymer chain.

14. Composition according to Claim 13, characterized by the fact that the said groups have a quaternization value, per gram of polymer, at least equal to 0.05 cationic meg.

15. Composition according to any one of Claims 1 to 12, characterized by the fact that the said amine or quaternary ammonium groups are carried by a side substituent.

16. Composition according to the preceding claim, characterized by the fact that the polymer chain of the said cationic polymer is an acrylic, vinyl, siliconized, fluorinated or saccharide chain.

17. Composition according to any one of the preceding claims, characterized by the fact that it is provided in the form of a compacted powder, a cast powder, a lipstick, a nail varnish or an emulsion.

18. Composition according to any one of the preceding claims, characterized by the fact that it constitutes an anhydrous composition.

19. Process of cosmetic treatment intended for applying makeup to the skin or superficial body growths, characterized by the fact that a composition as defined in any one of Claims 1 and 3 to 18 is applied to the skin or superficial body growths.

20. Process of cosmetic treatment characterized by the fact that the composition as defined in any one of Claims 2 and 4 to 18 is applied to the nails.

## Patentansprüche

1. Kosmetische Zubereitung für die Haut oder Nägel sowie behaarte Stellen oder andere Epidermisbildungen mit einem Gehalt an einer Dispersion aus Festteilchen in einem Bindemittel, dadurch gekennzeichnet, daß mindestens ein Teil dieser Teilchen in diese Zubereitung in Form von Teilchen eingearbeitet werden, deren Oberfläche mit mindestens einem kationischen Polymer überzogen ist, wobei dieses kationische Polymer entweder in der Polymerkette oder in den seitlichen Substituenten primäre, sekundäre oder tertiäre Aminogruppen und/oder quaternäre Ammoniumgruppen enthält, wobei diese Teilchen weiterhin dadurch gekennzeichnet sind, daß das Bindemittel ein fettes Bindemittel ist.

2. Wasserfreie Nagellack-Zusammensetzung mit einem Gehalt an einer Dispersion aus Festteilchen in einem Bindemittel, dadurch gekennzeichnet, daß mindestens ein Teil dieser Teilchen in diese Zubereitung in Form von Teilchen eingearbeitet wird, deren Oberfläche mit mindestens einem kationischen Polymer überzogen ist, wobei dieses kationische Polymer entweder in der Polymerkette oder an den seitlichen Substituenten Einheiten mit primären, sekundären oder tertiären Aminogruppen und/oder quaternären Ammoniumgruppen enthält, und die Zubereitung weiterhin dadurch gekennzeichnet ist, daß das Bindemittel aus einer filmbildenden Polymerlösung und einem Weichmacher in einem geeignetem organischen Lösungsmittel besteht.

3. Kosmetische Zubereitung für die Haut oder für die Nägel sowie behaarte Stellen oder andere Epidermisbildungen mit einem Gehalt an einer Dispersion aus Festteilchen in einem Bindemittel, dadurch gekennzeichnet, daß mindestens ein Teil dieser Teilchen in diese Zubereitung in Form von Teilchen eingearbeitet wird, deren Oberfläche mit mindestens einem kationischen Polymer überzogen ist, wobei dieses kationische Polymer entweder in der Kette oder an den seitlichen Substituenten Einheiten mit quaterären Ammoniumgruppen enthält.

4. Zubereitung nach einem der vorangehenden Ansprüche, dadurch gekennzeichnet, daß die überzogenen Teilchen mineralische Trägerstoffe oder mineralische Pigmente aufweisen.

5. Zubereitung nach dem vorstehenden Anspruch, dadurch gekennzeichnet, daß die überzogenen Teilchen Trägerstoffe aufweisen, die aus den folgenden Substanzen ausgewählt sind: Calciumcarbonate, Silikate, wie zum Beispiel das Aluminiumsilikat oder Kaolin, Calciumsilikate, Natriumaluminiumsilikat, Magnesiumsilikat oder Talkum, Kaliumaluminiumsilikate oder Glimmer sowie hydratisiertes Magnesiumaluminiumsilikat, Sulfate, wie zum Beispiel das Bariumsulfat oder Calciumsulfat, ausgefällte oder flammendisperse Siliziumdioxide wie zum Beispiel Hydrogele und Aerogele der Kieselerde.

6. Zubereitung nach Anspruch 4, dadurch gekennzeichnet, daß die überzogenen Teilchen Pigmente aufweisen, welche aus den folgenden Substanzen ausgewählt sind:
weiße Pigmente, wie zum Beispiel Titandioxid oder Zinkoxid sowie farbige Pigmente wie zum Beispiel gefärbte Eisenoxide natürlichen oder synthetischen Ursprungs von schwarzer, roter und gelber Farbe, gegebenenfalls hydratisierte grüne Chromoxide, Preußisch Blau, Natriumaluminuimsulfosilikate sowie Ultramarinpigmente, Kobaltaluminat oder Kobaltblau sowie Manganviolett.

7. Zubereitung nach Anspruch 4, dadurch gekennzeichnet, daß die überzogenen Teilchen perlmuttglänzende Pigmente, wie zum Beispiel Titanglimmerverbindungen sowie Wismutoxychlorid enthalten.

8. Zubereitung nach Anspruch 4, dadurch gekennzeichnet, das die überzogenen Teilchen Zink-, Cerium-, Zirkoniumpigmente oder deren Gemische enthalten.

9. Zubereitung nach einem der vorstehenden Ansprüche dadurch gekennzeichnet, daß das Gewichtsverhältnis an kationischen Polymeren in den überzogenen Teilchen mindestens 0,1 Gew.-% in bezug auf das Gesamtgewicht der überzogenen Teilchen beträgt.

10. Zubereitung nach dem vorstehenden Anspruch, dadurch gekennzeichnet, daß das Verhältnis weniger als 10 %, vorzugsweise weniger als 8 %, beträgt.

11. Zubereitung nach einem der vorstehenden Ansprüche, dadurch gekennzeichnet, daß die überzogenen Teilchen einzig und allein mit einem oder mehreren kationischen Polymeren überzogen sind.

12. Zubereitung nach einem der vorstehenden Ansprüche, dadurch gekennzeichnet, daß das kationische Polymer mindestens 10 Gew.-% an Einheiten mit primären, sekundären, tertiären Aminogruppen und/oder quaternären Ammoniumgruppen enthält.

13. Zubereitung nach einem der vorstehenden Ansprüche, dadurch gekennzeichnet, daß das kationische Polymer Aminogruppen oder quaternäre Ammoniumgruppen enthält, welche an der Bildung der Polymerkette teilhaben.

14. Zubereitung nach Anspruch 13, dadurch gekennzeichnet, daß der Quaternisierungsgrad der Gruppen mindestens oder gleich 0,05 Milliäquivalente Kation pro Gramm Polymer beträgt.

15. Zubereitung nach einem der Ansprüche 1 bis 12, dadurch gekennzeichnet, daß sich die Aminogruppen oder quaternären Ammoniumgruppen an einem seitlichen Substituenten befinden.

16. Zubereitung nach dem vorstehenden Anspruch, dadurch gekennzeichnet, daß die Polymerkette des kationischen Polymeren eine Acryl-, Vinyl-, silikonisierte, fluorierte oder verzuckerte Kette darstellt.

17. Zubereitung nach einem der vorstehenden Ansprüche, dadurch gekennzeichnet, daß sie in Form eines Kompaktpuders, eines in Form gegossenen Puders, eines Lippenstifftes, eines Nagellacks oder einer Emulsion vorliegt.

18. Zubereitung nach einem der vorstehenden Ansprüche, dadurch gekennzeichnet, daß sie aus einer wasserfreien Zusammensetzung besteht.

19. Verfahren zur kosmetischen Behandlung, die zum Schminken der Haut oder der Nägel bzw. behaarter Stellen oder anderer Epidermisbildungen bestimmt und dadurch gekennzeichnet ist, daß auf die Haut oder die übrigen vorstehend bezeichneten Stellen eine Zubereitung appliziert wird, wie sie in einem der Ansprüche 1 und 3 bis 18 definiert ist.

20. Verfahren zur kosmetischen Behandlung, dadurch gekennzeichnet, daß auf die Nägel eine Zubereitung appliziert wird, wie sie in einem der Ansprüche 2 und 4 bis 18 definiert ist.
